# NEW EUROPEAN PATENT SPECIFICATION

(11) **EP 1 808 496 B2**
(45) Date of publication and mention of the opposition decision: **22.12.2021**
(45) Mention of the grant of the patent: 08.08.2012
(21) Application number: 07008271.4
(22) Date of filing: 30.01.2002
(51) Int. Cl.: C12Q 1/6837, B01J 19/00

(54) **Methods of sequencing polynucleotide arrays, and preparation methods therefor**
Verfahren zum Sequenzieren von Polynukleotid-Arrays sowie Herstellungsmethoden dafür
Méthodes de séquençage de puces à polynucléotides, ainsi que méthodes pour leur fabrication

(30) Priority: 30.01.2001 US 771708
(43) Date of publication of application: 18.07.2007
(62) Divisional of application: 02712025.2
(73) Proprietor: Illumina Cambridge Limited, Cambridge CB21 6DF (GB)
(72) Inventor: Barnes, Colin, Great Abington Cambridge Cambridgeshire CB21 6DF (GB); Balasubramanian, Shankar, Great Abington Cambridge Cambridgeshire CB21 6DF (GB); Klenerman, David, Great Abington Cambridge Cambridgeshire CB21 6DF (GB); Osborne, Mark, Allen, Great Abington Cambridge Cambridgeshire CB21 6DF (GB)
(74) Representative: Stratagem IPM Limited

(56) References cited:
- WO-A-00/06770
- WO-A-00/36152
- WO-A-01/57249
- WO-A-02/02813
- WO-A-98/44151
- WO-A1-02/072892
- SE-A- 9 500 589
- US-A- 5 654 413
- US-A- 6 130 044
- US-A- 6 159 695
- AILEEN CONSTANS: "To Dream the Not-So-Impossible Genomics Dream" THE SCIENTIST, vol. 16, no. 20, 14 October 2002 (2002-10-14), page 53, XP002219824 Philadelphia, PA, US

## Description

### Field of the Invention

This invention relates to fabricated arrays of polynucleotides, and to their analytical applications.

### Background of the Invention

Advances in the study of molecules have been led, in part, by improvement in technologies used to characterise the molecules or their biological reactions. In particular, the study of nucleic acids, DNA and RNA, has benefited from developing technologies used for sequence analysis and the study of hybridisation events.

An example of the technologies that have improved the study of nucleic acids, is the development of fabricated arrays of immobilised nucleic acids. These arrays typically consist of a high-density matrix of polynucleotides immobilised onto a solid support material. Fodor et al., Trends in Biotechnology (1994) 12: 19-26, describes ways of assembling the nucleic acid arrays using a chemically sensitised glass surface protected by a mask, but exposed at defined areas to allow attachment of suitably modified nucleotides. Typically, these arrays may be described as "many molecule" arrays, as distinct regions are formed on the solid support comprising a high density of one specific type of polynucleotide.

An alternative approach is described by Schena et al., Science (1995) 270:467-470, where samples of DNA are positioned at predetermined sites on a glass microscope slide by robotic micropipetting techniques. The DNA is attached to the glass surface along its entire length by non-covalent electrostatic interactions. However, although hybridisation with complementary DNA sequences can occur, this approach may not permit the DNA to be freely available for interacting with other components such as polymerase enzymes, DNA-binding proteins etc.

Methods of analysing single nucleic acid molecules are known in the art, for example WO00/36152, WO00/06770 and SE-A-9500589. WO-A-96/27025 is a general disclosure of single molecule arrays. Although sequencing procedures are disclosed, there is little description of the applications to which the arrays can be applied. There is also only a general discussion on how to prepare the arrays.

### Summary of the Invention

The present invention relates to methods of sequencing, methods for the production of an array, and uses of a device comprising a high density array, as defined in the claims.

device comprises a high density array of relatively short molecules and relatively long polynucleotides immobilised on the surface of a solid support, wherein the polynucleotides are at a density that permits individual resolution ofthose parts that extend beyond the relatively short molecules. In this aspect, the shorter molecules help to control the density ofthe polynucleotides, providing a more uniform array of single polynucleotide molecules, thereby improving imaging. The small molecules may also prevent non-specific binding of reagents to the solid support, and therefore reduce background interference. For example, in the context of a polymerase reaction to incorporate nucleoside triphosphates onto a strand complementary to a long polynucleotide, the small molecules prevent the polymerase and nucleosides from attaching to the solid support surface, which may otherwise interfere with the imaging process.

The shorter molecules may also ensure that each polynucleotide is maintained upright, preventing the polynucleotides from interacting lengthwise with the solid support, which may otherwise prevent efficient interaction with a reagent, e.g. a polymerase. This may also prevent the fluorophore being quenched by the surface and therefore lead to more accurate imaging of the single polynucleotides.

According to a second aspect of the invention, a method for the production of an array of polynucleotides which are at a density that permits individual resolution, comprises arraying on the surface of a solid support, a mixture of relatively short molecules and relatively long polynucleotides, wherein the short molecules are arrayed in an amount in excess of the polynucleotides.

The arrays ofthe present invention comprise what are effectively single analysable polynucleotides. This has many important benefits for the study ofthe polynucleotides and their interaction with other biological molecules. In particular, fluorescence events occurring on each polynucleotide can be detected using an optical microscope linked to a sensitive detector, resulting in a distinct signal for each polynucleotide.

When used in a multi-step analysis of a population of single polynucleotides, the phasing problems that are encountered using high density (multi-molecule) arrays of the prior art, can be reduced or removed. Therefore, the arrays also permit a massively parallel approach to monitoring fluorescent or other events on the polynucleotides. Such massively parallel data acquisition makes the arrays extremely useful in a wide range of analysis procedures which involve the screening/characterising ofheterogeneous mixtures of polynucleotides.

The preparation ofthe arrays requires only small amounts of polynucleotide sample and other reagents, and can be carried out by simple means.

### Description of the Drawings

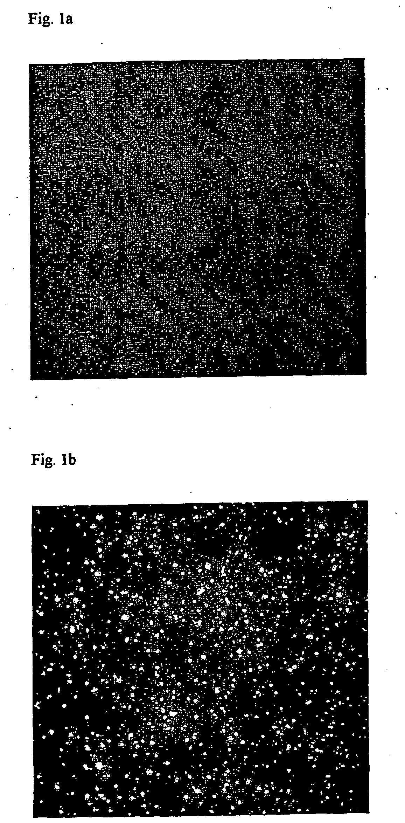

Figures 1a and b are images of a single polynucleotide array, where single polynucleotides are indicated by the detection of a fluorescent signal generated on the array.

### Description of the Invention

The single polynucleotide array devices are fabricated to include a "monolayer" of relatively short molecules that coat the surface of a solid support material and provide a flexible means to control the density of the single polynucleotides and optionally to prevent non-specific binding of reagents to the solid support.

In the context of the present invention, the terms "relatively short" and "relatively long" should be interpreted to mean that the "relatively long" polynucleotides extend above the "relatively short" molecules when arrayed.

The single polynucleotides immobilised onto the surface of a solid support should be capable of being resolved by optical means. This means that, within the resolvable area of the particular imaging device used, there must be one or more distinct images each representing one polynucleotide. Typically, the polynucleotides of the array are resolved using a single molecule fluorescence microscope equipped with a sensitive detector, e.g. a charge-coupled device (CCD). Each polynucleotide of the array may be imaged simultaneously or, by scanning the array, a fast sequential analysis can be performed.

The polynucleotides of the array are typically DNA or RNA, although nucleic acid mimics, e.g. PNA or 2'-O-Meth-RNA, are within the scope of the invention. The polynucleotides are formed on the array to allow interaction with other molecules. It is therefore important to immobilise the polynucleotides so that the portion of the polynucleotide not physically attached to solid support is capable of being interrogated. In some applications all the polynucleotides in the single array will be the same, and may be used to capture molecules that are largely distinct. In other applications, the polynucleotides on the array may all, or substantially all, be different, e.g. less than 50%, preferably less than 30% of the polynucleotides will be the same.

The term "single molecule" is used herein to distinguish from high density multi-molecule (polynucleotide) arrays in the prior art, which may comprise distinct clusters of many polynucleotides of the same type.

The term "individually resolved" is used herein to indicate that, when visualised, it is possible to distinguish one polynucleotide on the array from its neighbouring polynucleotides. Visualisation may be effected by the use of reporter labels, e.g. fluorophores, the signal of which is individually resolved. There may be some polynucleotides present on the solid support that are not capable of being individually resolved, however, these can be discounted during imaging, provided that the majority of the polynucleotides can be resolved at the single molecule level.

The term "interrogate" is used herein to refer to any interaction of the arrayed polynucleotide with any other molecule, e.g. with a polymerase or nucleoside triphosphate.

However, the present invention makes use of arrays with a high density of 10⁶-10⁹ single polynucleotides per cm². Preferably, the density is at least 10⁷/cm² and typically up to 10⁸/cm². These high density arrays are in contrast to other arrays which may be described in the art as "high density" but which are not necessarily as high and/or which do not allow single molecule resolution.

The shorter molecules will typically be present on the array at much higher density, to coat the remaining surface of the solid support. The shorter molecules may therefore be brought into contact with the solid support at an excess concentration. The small molecules are at a density greater than 10¹² molecules/cm².

Using the methods of the present invention, it may be possible to image at least 10⁵ or 10⁸ polynucleotides/cm2, preferably at least 10⁷ polynucleotides/cm2. Fast sequential imaging may be achieved using a scanning apparatus; shifting and transfer between images may allow higher numbers of polynucleotides to be imaged.

The extent of separation between the individual polynucleotides on the array will be determined, in part, by the particular technique used to resolve the individual polynucleotide. Apparatus used to image molecular arrays are known to those skilled in the art. For example, a confocal scanning microscope may be used to scan the surface of the array with a laser to image directly a fluorophore incorporated on the individual polynucleotide by fluorescence. Alternatively, a sensitive 2-D detector, such as a charge-coupled device, can be used to provide a 2-D image representing the individual polynucleotides on the array.

Resolving single polynucleotides on the array with a 2-D detector can be done if, at 100 x magnification, adjacent polynucleotides are separated by a distance of approximately at least 250nm, preferably at least 300nm and more preferably at least 350nm. It will be appreciated that these distances are dependent on magnification, and that other values can be determined accordingly, by one of ordinary skill in the art.

Other techniques such as scanning near-field optical microscopy (SNOM) are available which are capable of greater optical resolution, thereby permitting more dense arrays to be used. For example, using SNOM, adjacent polynucleotides may be separated by a distance of less than 100nm, e.g. 10nm. For a description of scanning near-field optical microscopy, see Moyer et al., Laser Focus World (1993) 29(10).

An additional technique that may be used is surface-specific total internal reflection fluorescence microscopy (TIRFM); see, for example, Vale et al., Nature, (1996) 380: 451-453). Using this technique, it is possible to achieve wide-field imaging (up to 100 µm x 100 µm) with single molecule sensitivity. This may allow arrays of greater than 107 resolvable polynucleotides per cm2 to be used.

Additionally, the techniques of scanning tunnelling microscopy (Binnig et al., Helvetica Physica Acta (1982) 55:726-735) and atomic force microscopy (Hansma et al., Ann. Rev. Biophys. Biomol. Struct. (1994) 23:115-139) are suitable for imaging arrays. Other devices which do not rely on microscopy may also be used, provided that they are capable of imaging within discrete areas on a solid support.

The devices comprise immobilised polynucleotides and other immobilised molecules. The other molecules are relatively short compared to the polynucleotides and are used to control the density ofthe polynucleotides. They may also prevent non-specific attachment of reagents, e.g. nucleoside triphosphates, with the solid support, thereby reducing background interference. In one embodiment, the shorter molecules are also polynucleotides. However, many different molecules may be used, e.g. peptides, proteins, polymers and synthetic chemicals, as will be apparent to the skilled person. The preferred molecules are organic molecules that contain groups that can react with the surface of a solid support.

Preparation of the devices may be carried out by first preparing a mixture of the relatively long polynucleotides and of the relatively short molecules. Usually, the concentration of the latter will be in excess of that of the long polynucleotides. The mixture is then placed in contact with a suitably prepared solid support, to allow immobilisation to occur.

Single polynucleotides may be immobilised to the surface of a solid support by any known technique, provided that suitable conditions are used to ensure adequate separation. Density of the polynucleotide molecules may be controlled by dilution. The gaps between the polynucleotides can be filled in with short molecules (capping groups) that may be small organic molecules or may be polynucleotides of different composition. The formation of the array of individually resolvable "longer" polynucleotides permits interrogation of those polynucleotides that are different from the bulk of the molecules.

Suitable solid supports are available commercially, and will be apparent to the skilled person. The supports may be manufactured from materials such as glass, ceramics, silica and silicon. Supports with a gold surface may also be used. The supports usually comprise a flat (planar) surface, or at least a structure in which the polynucleotides to be interrogated are in the same plane. Any suitable size may be used. For example, the supports might be of the order of 1-10 cm in each direction.

Immobilisation may be by specific covalent or non-covalent interactions. Covalent attachment is preferred. Immobilisation of a polynucleotide will be carried out at either the 5' or 3' position, so that the polynucleotide is attached to the solid support at one end only. However, the polynucleotide may be attached to the solid support at any position along its length, the attachment acting to tether the polynucleotide to the solid support; this is shown for the hairpin constructs, described below. The immobilised (relatively long) polynucleotide is then able to undergo interactions with other molecules or cognates at positions distant from the solid support. Immobilisation in this manner results in well separated long polynucleotides. The advantage of this is that it prevents interaction between neighbouring long polynucleotides on the array, which may hinder interrogation of the array.

Suitable methods for forming the devices with relatively short molecules and relatively long polynucleotides will be apparent to the skilled person, based on conventional chemistries. The aim is to produce a highly dense layer ofthe relatively short molecules, interspersed with the relatively large polynucleotides which are at a density that permits resolution of each single polynucleotide.

A first step in the fabrication of the arrays will usually be to functionalise the surface of the solid support, making it suitable for attachment of the molecules/polynucleotides. For example, silanes are known functional groups that have been used to attach molecules to a solid support material, usually a glass slide. The relatively short molecules and relatively long polynucleotides can then be brought into contact with the functionalised solid support, at suitable concentrations and in either separate or combined samples, to form the arrays.

In one preferred embodiment, the long polynucleotides and the short molecules each have the same reactive group that attaches to the solid support, or to an intermediary molecule.

In an alternative embodiment, the support surface may be treated with different functional groups, one of which is to react specifically with the relatively short molecules, and the other with the relatively long polynucleotides. Controlling the concentration of each functional group provides a convenient way to control the densities of the molecules/polynucleotides.

In a still further embodiment, the relatively short molecules are immobilised at high density onto the surface of the solid support. The molecules are capable of reacting with the polynucleotides (either directly or through an intermediate functional group) which can be brought into contact with the molecules at a suitable concentration to provide the required density. The polynucleotides are therefore immobilised on top of the monolayer of molecules. Those molecules that are not in contact with a polynucleotide may be reacted with a further molecule to block (or cap) the reactive site. This may be carried out before, during or after arraying the polynucleotides. The blocking (capping) group may itself be a relatively short polynucleotide.

Alternatively, only a minor proportion of the short molecules that are arrayed at high density on the solid support comprise a group that reacts with the polynucleotides; the majority are non-reactive. For example, the short molecules can be mixed silanes, a minor proportion ofwhich are reactive with a functional group on the polynucleotides, and the remaining silanes are unreactive and form the array of short molecules on the device. Therefore, controlling the concentration of the minor proportion of short molecules also controls the density of the polynucleotides.

In this embodiment, the short molecules may have been modified in solution prior to immobilisation on the array so that only a minor proportion contain a functional group that is capable of undergoing covalent attachment to a complementary functional group on the polynucleotides.

In a related embodiment, the short molecules are polynucleotides, and appropriate concentrations of both relatively long and relatively short polynucleotides are reacted with a functional group and then arrayed on the solid support, or to an intermediate molecule bound to the solid support.

Suitable functional groups will be apparent to the skilled person. For example, suitable groups include: amines, acids, esters, activated acids, acid halides, alcohols, thiols, disulfides, olefins, dienes, halogenated electrophiles and phosphorothioates. It is preferred if the group contains a silane.

The relatively small molecules may be any molecule that can provide a barrier against non-specific binding to the solid support.

Suitable small molecules may be selected based on the required properties of the surface and the existing functionality.

In a preferred embodiment, the molecules are silanes of type RₙSiX₍₄₋ₙ₎ (where R is an inert moiety that is displayed on the surface of the solid support and X is a reactive leaving group of type Cl or O-alkyl). The silanes include tetraethoxysilane, triethoxymethylsilane, diethoxydimethylsilane orglycidoxypropyltriethoxysilane, although many other suitable examples will be apparent to the skilled person.

In an embodiment of the invention, the short molecules act as surface blocks to prevent random polynucleotide association with the surface of the solid support. Molecules therefore require a group to react with the surface (which will preferably be the same functionality as used to attach the polynucleotide to the surface) and an inert group that will be defined by the properties required on the surface. In an embodiment, the surface is functionalised with an epoxide and the small molecule is glycine, although other compounds containing an amine group would suffice.

It is also preferred if the small molecule is hydrophilic and repels binding of anions. The molecule therefore may be acid, phosphate, sulfate, hydroxyl or polyol and may include polyethers such as PEG.

In one embodiment, the relatively short molecules are polynucleotides. These may be prepared using any suitable technique, including synthetic techniques known in the art. It may be preferable to use short polynucleotides that are immobilised to the solid support at one end and comprise, at the other end, a non-reactive group, e.g. a dideoxynucleotide: incapable of incorporating further nucleotides. The short polynucleotide may also be a hairpin construct, provided that it does not interact with a polymerase.

In one embodiment of the present invention, each relatively long polynucleotide of the array comprises a hairpin loop structure, one end of which comprises a target polynucleotide, the other end comprising a relatively short polynucleotide capable of acting as a primer in a polymerase reaction. This ensures that the primer is able to perform its priming function during a polymerase-based sequencing procedure, and is not removed during any washing step in the procedure. The target polynucleotide is capable of being interrogated.

The term "hairpin loop structure" refers to a molecular stem and loop structure formed from the hybridisation of complementary polynucleotides that are covalently linked. The stem comprises the hybridised polynucleotides and the loop is the region that covalently links the two complementary polynucleotides. Anything from a 5 to 25 (or more) base pair double-stranded (duplex) region may be used to form the stem. In one embodiment, the structure may be formed from a single-stranded polynucleotide having complementary regions. The loop in this embodiment may be anything from 2 or more non-hybridised nucleotides. In a second embodiment, the structure is formed from two separate polynucleotides with complementary regions, the two polynucleotides being linked (and the loop being at least partially formed) by a linker moiety. The linker moiety forms a covalent attachment between the ends ofthe two polynucleotides. Linker moieties suitable for use in this embodiment will be apparent to the skilled person. For example, the linker moiety may be polyethylene glycol (PEG).

If the short molecules are polynucleotides in a hairpin construct, it is possible to ligate the relatively long polynucleotides to a minor proportion of the hairpins either prior to or after arraying the hairpins on the solid support.

The arrays have many applications in methods which rely on the detection of biological or chemical interactions with polynucleotides. For example, the arrays may be used to determine the properties or identities of cognate molecules. Typically, interaction of biological or chemical molecules with the arrays are carried out in solution.

In particular, the arrays may be used in conventional assays which rely on the detection of fluorescent labels to obtain information on the arrayed polynucleotides. The arrays are particularly suitable for use in multi-step assays where the loss of synchronisation in the steps was previously regarded as a limitation to the use of arrays. The arrays may be used in conventional techniques for obtaining genetic sequence information. Many of these techniques rely on the stepwise identification of suitably labelled nucleotides, referred to in US-A-5634413 as "single base" sequencing methods.

In an embodiment of the invention, the sequence of a target polynucleotide is determined in a similar manner to that described in US-A-5634413, by detecting the incorporation of nucleotides into the nascent strand through the detection of a fluorescent label attached to the incorporated nucleotide. The target polynucleotide is primed with a suitable primer (or prepared as a hairpin construct which will contain the primer as part of the hairpin), and the nascent chain is extended in a stepwise manner by the polymerase reaction. Each of the different nucleotides (A, T, G and C) incorporates a unique fluorophore at the 3' position which acts as a blocking group to prevent uncontrolled polymerisation. The polymerase enzyme incorporates a nucleotide into the nascent chain complementary to the target, and the blocking group prevents further incorporation of nucleotides. The array surface is then cleared of unincorporated nucleotides and each incorporated nucleotide is "read" optically by a charge-coupled device using laser excitation and filters. The 3' -blocking group is then removed (deprotected), to expose the nascent chain for further nucleotide incorporation.

Because the array consists of distinct optically resolvable polynucleotides, each target polynucleotide will generate a series of distinct signals as the fluorescent events are detected. Details of the full sequence are then determined.

Other suitable sequencing procedures will be apparent to the skilled person. In particular, the sequencing method may rely on the degradation of the arrayed polynucleotides, the degradation products being characterised to determine the sequence.

An example of a suitable degradation technique is disclosed in WO-A- 95/20053, whereby bases on a polynucleotide are removed sequentially, a predetermined number at a time, through the use of labelled adaptors specific for the bases, and a defined exonuclease cleavage.

A consequence of sequencing using non-destructive methods is that it is possible to form a spatially addressable array for further characterisation studies, and therefore non-destructive sequencing may be preferred. In this context, the term "spatially addressable" is used herein to describe how different molecules may be identified on the basis of their position on an array.

Once sequenced, the spatially addressed arrays may be used in a variety of procedures which require the characterisation of individual molecules from heterogeneous populations.

The following Examples illustrate the invention, with reference to the accompanying drawings.

### Example I

Glass slides were cleaned with decon 90 for 12 h at room temperature prior to use, rinsed with water, EtOH and dried. A solution of glycidoxypropyltrimethoxysilane (0.5 ml) and mercaptopropyltrimethoxysilane (0.0005 ml) in acidified 95% EtOH (50 ml) was mixed for 5 min. The clean, dried slides were added to this mixture and left for 1 h at room temperature rinsed with EtOH, dried and cured for 1 h at 1000C. Maleimide modified DNA was prepared from a solution of amino-DNA (5'-Cy3-CtgCTgAAgCgTCggCAggT-heg-aminodT-heg-ACCTgCCgACgCT; SEQ ID NO.1) (10 µM, 100 µL) and N-[y-Maleimidobutryloxy]succinimide ester (GMBS); (Pierce) (1 mM) in DMF/diisopropylethylamine (DIPEA)/water (89/1/10) for 1 h at room temperature. The excess cross-linker was removed using a size exclusion cartridge (NAPS) and the eluted DNA freeze-dried in aliquots and freshly diluted prior to use. An aliquot of the maleimide-GMBS-DNA (100 nM) was placed on the thiol surface in 50 mM potassium phosphate/1 mM EDTA (pH 7.6) and left for 12 h at room temperature prior to washing with the same buffer.

The slide was inverted so that the chamber coverslip contacted the objective lens of an inverted microscope (Nikon TE200) via an immersion oil interface. A 60° fused silica dispersion prism was optically coupled to the back of the slide through a thin film of glycerol. Laser light was directed at the prism such that at the glass/sample interface it subtended an angle of approximately 68° to the normal of the slide and subsequently underwent Total Internal Reflection (TIR). Fluorescence from the surface produced by excitation with the surface specific evanescent wave generated by TIR was collected by the objective lens of the microscope and imaged onto an intensified charged coupled device (ICCD) camera (Pentamax, Princeton Instruments).

Images were recorded using a combination of a 532 Nd:YAG laser with a 580DF30 emission filter (Omega optics), with an exposure of 500 ms and maximum camera gain and a laser power of 50 mW at the prism.

The presence of glycidoxypropyltrimethoxysilane gave improved results (Fig. 1 a) compared to a control carried out in the absence of glycidoxypropyltrimethoxysilane.

### Example 2

Slides were cleaned with decon 90 for 12 h prior to use and rinsed with water, EtOH and dried. A solution of tetraethoxysilane (0.7 mL) and N-(3-triethoxysilylpropyl)bromoacetamide (0.0007 mL) in acidified 95% EtOH (35 mL) was mixed for 5 min. The clean, dried slides were added to this mixture and left for 1 h at room temperature, rinsed with EtOH, dried and cured for 1 h at 100°C. Phosphorothioate modified DNA (5'-TMR-TACCgTCgACgTCgACgCTggCgAgCgTgCTgCggTlsTsTsTsT ACCgCAgCACgCTCgCCAgCg; SEQ ID NO. 2) where s = phosphorothioate (100 pM, 100 µL) in sodium acetate (30 mM, pH 4.5) was added to the surface and left for 1 h at room temperature. The slide was washed with a buffer containing 50 mM Tris/1 mM EDTA.

Imaging was performed as described in Example 1 and a good dispersion of single molecules was seen (Fig. 1b).

### Example 3

Slides were cleaned with decon 90 for 12 h prior to use and rinsed with water, EtOH and dried. A solution of glycidoxypropyltrimethoxysilane (0. 5 mL) in acidified 95% EtOH was prepared and the cleaned slides placed in the solution for 1 h, rinsed with EtOH and dried. Amino modified DNA (5'-Cy3-CTgCTgAAgCgTCggCAggT-heg-aminodT-heg-ACCTgCCgACgCT; SEQ ID NO. 1) (1 µM, 100 µL) was placed on the surface and left for 12 h at room temperature. The slide was washed with a solution of 1 mM glycine at pH 9 for 1 h and flushed with 50 mM potassium phosphate/1 mM EDTA (pH 7:6). A good dispersion of coupled single molecules was seen by TIR microscopy, as described in Example 1.

The slide was then exposed to a mixture containing Cy5-dUTP (20 µM) and T4 exo-polymerase (250 nM) and Tris (40 mM), NaCl (10 mM), MgCl₂ (4 mM), DTT (2 mM), potassium phosphate (1 mM), BSA (0.2 mgs/ml) 100 µL) at room temperature for 10 min. and then flushed with Tris/EDTA buffer.

Imaging was performed using a pumped dye laser at 630 nm with a 670DF40 emission filter at 40 mW laser power using the TIR setup as described. A lower level of non-specific trisphosphate binding was seen in the case using glycine, than in a control not treated with glycine.

## Claims

1. A method of sequencing an array of single polynucleotides wherein the array has a density of from 10⁶ to 10⁹ single polynucleotides per cm² and a higher density of greater than 10¹² molecules/cm² of relatively short molecules, whereby those parts of the single polynucleotides that extend beyond the relatively short molecules can be individually resolved by optical means, wherein the sequencing is carried out by the stepwise identification of fluorescently labelled nucleotides incorporated onto a strand complementary to the single polynucleotides and wherein the identification is achieved by scanning the array using a sequential scanning apparatus which shifts between images.

2. A method according to claim 1, wherein the device has a density of from 10⁷ to 10⁸ polynucleotides per cm².

3. A method according to any one of claims 1 to 2 wherein the relatively short molecules are polynucleotides.

4. A method according to any one of claims 1 to 3 wherein less than 50% of the relatively long polynucleotides on the array are the same.

5. A method according to claim 4 wherein less than 30% of the relatively long polynucleotides on the array are the same.

6. A method according to claim 5 wherein all the relatively long polynucleotides on the array are different.

7. A method according to claims 1-6 wherein the imaging is total internal reflection fluorescence microscopy.

8. A method according to any one of claims 1 to 7 wherein the high density array of relatively short molecules and relatively long polynucleotides is prepared by arraying on the surface of a solid support a mixture of relatively long polynucleotides and relatively short molecules, wherein the short molecules are in excess of the polynucleotides.

9. A method according to claim 8 wherein the polynucleotides and short molecules are brought into contact with the solid support in a single composition.

10. A method according to claim 8 wherein the short molecules and the long polynucleotides are arrayed separately, with the short molecules being brought into contact with the solid support first.

11. A method for the production of an array of polynucleotides which are at a density of 10⁶ to 10⁹ individually resolvable polynucleotides per cm², comprising arraying on the surface of a solid support a mixture of relatively short molecules and relatively long polynucleotides, wherein the relatively short molecules are at a density of greater than 10¹² molecules/cm² and wherein the relatively short molecules and the relatively long polynucleotides are arrayed separately, with the relatively short molecules being brought into contact with the solid support first.

12. A method according to claim 11 which comprises arraying the relatively short molecules on the surface of a solid support and reacting said relatively short molecules with relatively long polynucleotides directly or through an intermediate functional group, wherein the short molecules are immobilised at high density and the relatively long polynucleotides are brought into contact with the short molecules at a suitable concentration to provide the required density of relatively long polynucleotides.

13. A method according to claim 11 which comprises the following steps:
i) functionalising the solid support,
ii) arraying the relatively short molecules on the surface of the solid support, and
iii) separately bringing the relatively long polynucleotides into contact with the functionalised solid support.

## Patentansprüche

1. Verfahren zum Sequenzieren einer Anordnung von einzelnen Polynukleotiden, wobei die Anordnung eine Dichte von 10⁶ bis 10⁹ einzelnen Polynukleotiden pro cm² und eine höhere Dichte von mehr als 10¹² Molekülen/cm² von relativ kurzen Molekülen aufweist, wodurch diese Teile der einzelnen Polynukleotide, die sich über die relativ kurzen Moleküle hinaus erstrecken, individuell durch optische Mittel aufgelöst werden können, wobei das Sequenzieren durch die schrittweise Identifizierung von fluoreszenzmarkierten Nukleotiden durchgeführt wird, die an einen Strang komplementär zu den einzelnen Polynukleotiden eingebunden sind und wobei die Identifizierung erreicht wird, indem die Anordnung unter Verwendung einer sequentiellen Abtastvorrichtung, die zwischen Bildern wechselt, abgetastet wird.

2. Verfahren nach Anspruch 1, wobei die Vorrichtung eine Dichte von 10⁷ bis 10⁸ Polynukleotiden pro cm² aufweist.

3. Verfahren nach einem der Ansprüche 1 bis 2, wobei die relativ kurzen Moleküle Polynukleotide sind.

4. Verfahren nach einem der Ansprüche 1 bis 3, wobei weniger als 50 % der relativ langen Polynukleotide auf der Anordnung gleich sind.

5. Verfahren nach Anspruch 4, wobei weniger als 30 % der relativ langen Polynukleotide auf der Anordnung gleich sind.

6. Verfahren nach Anspruch 5, wobei alle der relativ langen Polynukleotide auf der Anordnung unterschiedlich sind.

7. Verfahren nach Anspruch 1-6, wobei die Bildgebung Totalreflexions-Fluoreszenzmikroskopie ist.

8. Verfahren nach einem der Ansprüche 1 bis 7, wobei die Anordnung mit hoher Dichte aus relativ kurzen Molekülen und relativ langen Polynukleotiden hergestellt wird, indem auf der Oberfläche eines festen Trägers eine Mischung aus relativ langen Polynukleotiden und relativ kurzen Molekülen angeordnet wird, wobei die kurzen Moleküle die Polynukleotide übersteigen.

9. Verfahren nach Anspruch 8, wobei die Polynukleotide und die kurzen Moleküle in einer einzelnen Zusammensetzung mit dem festen Träger in Kontakt gebracht werden.

10. Verfahren nach Anspruch 8, wobei die kurzen Moleküle und die langen Polynukleotide separat angeordnet werden, wobei die kurzen Moleküle zuerst mit dem festen Träger in Kontakt gebracht werden.

11. Verfahren zur Herstellung einer Anordnung von Polynukleotiden, die bei einer Dichte von 10⁶ bis 10⁹ individuell auflösbaren Polynukleotiden pro cm² sind, umfassend das Anordnen einer Mischung aus relativ kurzen Molekülen und relativ langen Polynukleotiden auf der Oberfläche eines festen Trägers, wobei die relativ kurzen Moleküle bei einer Dichte von mehr als 10¹² Molekülen/cm² sind und wobei die relativ kurzen Moleküle und die relativ langen Polynukleotide separat angeordnet werden, wobei die relativ kurzen Moleküle zuerst mit dem festen Träger in Kontakt gebracht werden.

12. Verfahren nach Anspruch 11, welches das Anordnen der relativ kurzen Moleküle auf der Oberfläche eines festen Trägers und das Reagieren der relativ kurzen Moleküle mit relativ langen Polynukleotiden direkt oder durch eine funktionelle Zwischengruppe umfasst, wobei die kurzen Moleküle bei hoher Dichte immobilisiert werden und die relativ langen Polynukleotide mit den kurzen Molekülen bei einer geeigneten Konzentration in Kontakt gebracht werden, um die erforderliche Dichte von relativ langen Polynukleotiden bereitzustellen.

13. Verfahren nach Anspruch 11, das die folgenden Schritte umfasst:
i) Funktionalisieren des festen Trägers,
ii) Anordnen der relativ kurzen Moleküle auf der Oberfläche des festen Trägers, und
iii) separates Inkontaktbringen der relativ langen Polynukleotide mit dem funktionalisierten festen Träger.

## Revendications

1. Procédé de séquençage d'un réseau de polynucléotides simples, dans lequel le réseau a une densité de 10⁶ à 10⁹ polynucléotides simples par cm² et une densité plus élevée supérieure à 10¹² molécules/cm² de molécules relativement courtes, de sorte que les parties des polynucléotides simples qui s'étendent au-delà des molécules relativement courtes peuvent être résolues individuellement par un moyen optique, le séquençage étant effectué par l'identification progressive de nucléotides marqués par fluorescence incorporés sur un brin complémentaire des polynucléotides simples, et l'identification étant réalisée par balayage du réseau au moyen d'un appareil de balayage séquentiel qui passe d'une image à l'autre.

2. Procédé selon la revendication 1, dans lequel le dispositif a une densité de 10⁷ à 10⁸ polynucléotides par cm².

3. Procédé selon l'une quelconque des revendications 1 à 2, dans lequel les molécules relativement courtes sont des polynucléotides.

4. Procédé selon l'une quelconque des revendications 1 à 3, dans lequel moins de 50 % des polynucléotides relativement longs sur le réseau sont identiques.

5. Procédé selon la revendication 4, dans lequel moins de 30 % des polynucléotides relativement longs sur le réseau sont identiques.

6. Procédé selon la revendication 5, dans lequel tous les polynucléotides relativement longs sur le réseau sont différents.

7. Procédé selon les revendications 1-6, dans lequel l'imagerie est la microscopie par fluorescence à réflexion totale interne.

8. Procédé selon l'une quelconque des revendications 1 à 7, dans lequel le réseau à haute densité de molécules relativement courtes et de polynucléotides relativement longs est préparé par mise en réseau sur la surface d'un support solide d'un mélange de polynucléotides relativement longs et de molécules relativement courtes, dans lequel les molécules courtes excèdent les polynucléotides.

9. Procédé selon la revendication 8, dans lequel les polynucléotides et les molécules courtes sont mis en contact avec le support solide dans une composition simple.

10. Procédé selon la revendication 8, dans lequel les molécules courtes et les polynucléotides longs sont mis en réseau séparément, les molécules courtes étant mises en contact avec le support solide en premier.

11. Procédé pour la production d'un réseau de polynucléotides qui sont à une densité de 10⁶ à 10⁹ polynucléotides individuellement séparables par cm², comprenant la mise en réseau, sur la surface d'un support solide, d'un mélange de molécules relativement courtes et de polynucléotides relativement longs, où les molécules relativement courtes sont à une densité supérieure à 10¹² molécules/cm² et où les molécules relativement courtes et les polynucléotides relativement longs sont mis en réseau séparément, les molécules relativement courtes étant mises en contact avec le support solide en premier.

12. Procédé selon la revendication 11, qui comprend la mise en réseau des molécules relativement courtes sur la surface d'un support solide et la réaction desdites molécules relativement courtes avec les polynucléotides relativement longs directement ou via un groupe fonctionnel intermédiaire, où les molécules courtes sont immobilisées à haute densité et les polynucléotides relativement longs sont mis en contact avec les molécules courtes à une concentration appropriée pour fournir la densité requise de polynucléotides relativement longs.

13. Procédé selon la revendication 11, qui comprend les étapes suivantes :
i) la fonctionnalisation du support solide,
ii) la mise en réseau des molécules relativement courtes sur la surface du support solide, et
iii) la mise en contact séparée des polynucléotides relativement longs avec le support solide fonctionnalisé.


### SEQUENCE LISTING

<110> Solexa Limited
<120> The preparation of polynucleotide arrays
<130> P60489EP00
<160> 2
<170> PatentIn version 3.3
<210> 1
   <211> 34
   <212> DNA
   <213> Artificial sequence
<220>
   <223> Synthetic oligonucleotide
<220>
   <221> misc feature
   <222> (1)..(1)
   <223> m = cytosine with a fluorescent Cy3 group attached
<220>
   <221> misc feature
   <222> (21)..(21)
   <223> n = hexaethyleneglycol-aminodT-hexaethyleneglycol
<400> 1
   mtgctgaagc gtcggcaggt nacctgccga cgct 34
<210> 2
   <211> 62
   <212> DNA
   <213> Artificial sequence
<220>
   <223> Synthetic oligonucleotide
<220>
   <221> misc feature
   <222> (1)..(1)
   <223> n = thymine with a TMR group
<220>
   <221> misc feature
   <222> (37)..(40)
   <223> s = thymine modified with phosphorothioate
<400> 2
   naccgtcgac gtcgacgctg gcgagcgtgc tgcggtssss taccgcagca cgctcgccag      60
   cg                                                                     62